# EUROPÄISCHE PATENTSCHRIFT

(11) **EP 2 384 695 B1**
(45) Veröffentlichungstag und Bekanntmachung des Hinweises auf die Patenterteilung: **04.09.2019**
(21) Anmeldenummer: 11164495.1
(22) Anmeldetag: 02.05.2011
(51) Int. Cl.: A61B 5/00

(54) **Messsystem zur Analytbestimmung sowie Verfahren**
Measuring system for analyte detection and method
Système de mesure pour la détermination d'analyte et procédé

(30) Priorität: 03.05.2010 EP 10004640
(43) Veröffentlichungstag der Anmeldung: 09.11.2011
(73) Patentinhaber: Roche Diabetes Care GmbH, 68305 Mannheim (DE); F. Hoffmann-La Roche AG, 4070 Basel (CH)
(72) Erfinder: Krämer, Uwe, 68549 Ilvesheim (DE); Rasch-Menges, Jürgen, 68723 Schwetzingen (DE)
(74) Vertreter: Bittner, Thomas L.

(56) Entgegenhaltungen:
- DE-A1-102004 002 874
- JP-A- 2006 109 895
- JP-A- 2007 193 447
- US-A1- 2002 009 213
- US-A1- 2002 054 695
- US-A1- 2005 169 503
- US-A1- 2008 119 710

## Beschreibung

Die Erfindung betrifft ein Messsystem zur Analytbestimmung, insbesondere zur Blutzuckerbestimmung, sowie ein Verfahren zum Betreiben eines Messsystems zur Analytbestimmung.

### Hintergrund der Erfindung

Derartige Messsysteme sind dazu bestimmt, von einem oder mehreren Benutzern verwendet zu werden, um anhand der Analyse einer Körperflüssigkeitsprobe, insbesondere einer Urin-, Speichel-, Serum-, Plasma- oder Blutprobe des jeweiligen Benutzers einen Analytmesswert, zum Beispiel den Blutzuckerwert zu bestimmen. Hierzu verfügen die Messsysteme über eine Analyseeinrichtung zum experimentellen Analysieren der Blutprobe, um so einen Blutzuckermesswert zu ermitteln. Üblicherweise verfügen die Messsysteme über eine Steuereinrichtung, die mit Hardware- und Softwarekomponenten ausgestattet ist, um den Betrieb des Messsystems zu steuern, insbesondere auch in Abhängigkeit von erfassten Eingaben der Benutzer, die diese über eine oder mehrere Benutzerschnittstellen des Messsystems eingeben. Regelmäßig sind die Messsysteme zur Blutzuckerbestimmung als Handgeräte ausgeführt.

Aus dem Dokument US 2009/0138207 A1 ist ein tragbares Glukosemeter zur Bestimmung des Blutzuckerwertes einer Blutprobe bekannt, welches für eine Datenfernübertragung konfiguriert ist. Das bekannte Glukosemeter verfügt über eine Patentientenidentifizierungseinrichtung, die beispielsweise mit einem biometrischen Sensor gebildet sein kann. Eine ähnliche Vorrichtung ist auch im Dokument US 2007/0231209 A1 beschrieben.

Das Dokument US 2009/0010804 A1 offenbart eine Analysevorrichtung, zum Beispiel zum Analysieren einer Blutprobe, die in einer Ausführungsform mit einer Benutzeridentifikation auf Basis einer Fingerabdruckprüfung versehen ist. Eine Fingerabdruckverifikation ist weiterhin aus den Dokumenten US 7,116,805 sowie US 7,009,497 bekannt.

Das Dokument US 2007/0016104 A1 betrifft ein Glukosemeter, welches in einer Ausführungsform eine Benutzererkennung mit Hilfe der Auswertung eines Fingerabdrucks durchführt. Das Glukosemeter verfügt über ein Analysegerät zur Analyse von Blutproben sowie ein in das Analysegerät integriertes Benutzererkennungsverfahren. Beim Betrieb des Glukosemewerden Betriebsparameter nach einer erfolgreich durchgeführten Benutzererkennung dem erkannten Benutzer zugeordneten Betriebsparametern entsprechend eingestellt. Das Glukosemeter kann von mehreren Benutzern verwendet werden, weshalb es sich um eine Mehrbenutzerausfiihrung handelt. Die Durchführung der Benutzererkennung hat zur Folge, dass nichtautorisierte Personen von der Benutzung des Glukosemeters ausgeschlossen sind.

Das Dokument JP 2007 193447 A offenbart ein System zum Bestimmen biologischer Daten eines Patienten.

Das Dokument US 2002/009213 A1 betrifft ein Verfahren zur Durchführung einer biometrischen Identifikation.

Das Dokument US 2008/119710 A1 beschreibt ein medizinisches Gerät mit einem beschränken Zugriff.

Das Dokument JP 2006 109895 A betrifft ein Messgerät für eine Blutprobenbestimmung. Aus dem Dokument US 2002/054695 A1 ist ein Touchpad bekannt, welches in Abhängigkeit eines Fingerabdruckbildes als Zeigereinrichtung oder als Fingerabdrucksensor betrieben werden kann.

### Zusammenfassung der Erfindung

Aufgabe der Erfindung ist es, ein verbessertes Messsystem zur Analytbestimmung sowie ein Verfahren zum Betreiben des Messsystems anzugeben, mit denen die individuelle Benutzung des Messsystems durch eine oder mehrere Personen erreicht wird und effizienter gestaltet ist sowie der Bedienkomfort insgesamt optimiert ist.

Diese Aufgabe wird erfindungsgemäß durch ein Messsystem zur Analytbestimmung nach dem unabhängigen Anspruch 1 sowie ein Verfahren zum Betreiben eines Messsystems zur Analytbestimmung nach dem unabhängigen Anspruch 5 gelöst. Vorteilhafte Ausgestaltungen der Erfindung sind Gegenstand von abhängigen Unteransprüchen.

Die Steuereinrichtung ist weiterhin konfiguriert, bei der ergänzenden Auswertung der erfassten Fingerabdruck-Sensorsignale einen Finger einer menschlichen Hand zu identifizieren und in Abhängigkeit vom identifizierten Finger diesem zugeordnete Steuersignale bereitzustellen. Es ist vorgesehen, dass Steuersignale, die Betriebsfunktionen des Messsystems zugeordnet sind, in Abhängigkeit vom erkannten Finger erzeugt werden. Beispielsweise kann vorgesehen sein, dass beim Erkennen des Zeigefingers des Benutzers die Funktion "Messbereitschafts-Betriebsart" ausgelöst wird. Wenn hingegen der Mittelfinger des Benutzers detektiert wird, bedeutet dies zum Beispiel, dass das Messsystem für eine weitere Benutzung gesperrt wird, also eine Betriebsart "Ende der aktuellen Nutzung" ausgelöst wird. Eine Messung und eine zugehörige Datenspeicherung sind dann beendet. In dem Messsystem sind elektronische Informationen über den verschiedenen Fingern zugeordnete Funktionen gespeichert, so dass die entsprechenden Steuersignale beim Erkennen eines bestimmten Handfingers erzeugt werden können. Die fingerspezifische Festlegung kann benutzerbezogen oder losgelöst von bestimmten Benutzern erfolgen.

Die Erfindung hat gegenüber dem Stand der Technik den Vorteil, dass dem Benutzer eine individualisierte Nutzung des Messsystems auf einfache und bequeme Weise ermöglicht ist, nämlich mittels Berühren des Fingerabdruck-Sensors. Die Fingerabdruck-Sensorsignale werden ergänzend zu der vorgesehenen Benutzererkennung mittels der Steuereinrichtung ausgewertet, um hieraus Steuersignale abzuleiten, mit denen weitere Funktionen des Messsystems genutzt werden können. Die Fingerabdruck-Sensorsignale werden so in einer Art Zweitverwertung ausgewertet, d. h. ergänzend zu ihrer Auswertung in Verbindung mit der Benutzeridentifizierung. Die ergänzende Analyse der Fingerabdruck-Sensorsignale und die Bereitstellung der Steuersignale erfolgt frei von einer Berücksichtigung der elektronischen Informationen über die erfolgreiche Benutzererkennung. Dies kann bedeuten, dass die Steuersignale unabhängig von benutzerspezifischen Informationen erzeugt werden.

Bei der ergänzenden Auswertung können die zur Benutzeridentifizierung herangezogenen Fingerabdruck-Sensorsignale ganz oder teilweise ausgewertet werden. Es kann wahlweise vorgesehen sein, zusätzliche Fingerabdruck-Sensorsignale mit der Fingerabdruck-Sensoreinrichtung zu erfassen.

Eine Ausgestaltung kann vorsehen, dass das Messsystem mit einer Probennahmeeinrichtung gebildet ist. Diese umfasst dann in einer Ausgestaltung eine Stecheinrichtung zum Aufstechen der Haut für eine Blutprobennahme.

Mit Hilfe der Benutzeridentifizierung auf Basis der Fingerabdruck-Sensorsignale ist in einer Ausgestaltung die Ausbildung des Messsystems in Mehrbenutzerausführung ermöglicht.

Eine bevorzugte Weiterbildung der Erfindung sieht vor, dass die Steuereinrichtung weiterhin konfiguriert ist, bei erfolgreicher Benutzeridentifizierung Analytmesswerte, insbesondere Blutzuckerwerte, die mit Hilfe der Analyseeinrichtung für die aufgegebene Körperflüssigkeitsprobe, insbesondere Blutprobe bestimmt werden, dem identifizierten Benutzer zugeordnet für eine Datenspeicherung und / oder -ausgabe bereitzustellen. Die für einen identifizierten Benutzer bestimmten Analytmesswerte werden für eine Datenspeicherung und / oder -ausgabe so bereitgestellt, dass sie als dem identifizierten Benutzer zugeordnet erkennbar sind. Beispielsweise wird der einem Benutzer zugeordnete Datensatz mit einem den Benutzer identifizierenden Bezeichner versehen. In Verbindung mit der Datenspeicherung kann es vorgesehen sein, in der Steuereinrichtung eine Übersicht von Speicherbereichen vorzuhalten, die individuell unterschiedlichen Benutzern zugeordnet sind. Die einem identifizierten Benutzer zugeordneten Analytmesswerte werden dann in dem zugehörigen Speicherbereich abgelegt und können von dort wieder gelesen werden.

Bei einer zweckmäßigen Ausgestaltung der Erfindung kann vorgesehen sein, dass die Steuereinrichtung weiterhin konfiguriert ist, bei der ergänzenden Auswertung der erfassten Fingerabdruck-Sensorsignale in Abhängigkeit von den erfassten Fingerabdruck-Sensorsignalen Steuersignale zum Steuern eines Benutzermenüs bereitzustellen und das Benutzermenü den Steuersignalen entsprechend zu steuern. Das Benutzermenü wird dem Benutzer beispielsweise auf einem in das Messsystem integrierten Display angezeigt. Die Fingerabdruck-Sensorsig-Sensorsignale können in dieser Ausführungsform dahingehend ausgewertet werden, dass eine Navigation des Benutzers in dem dargestellten Benutzermenü erfasst wird. In einer Ausgestaltung ist die Fingerabdruck-Sensoreinrichtung deshalb als eine Art Track-Ball- oder Track-Pad-Vorrichtung ausgeführt. Es kann dem Benutzer auf diese Weise insbesondere auch die Möglichkeit gegeben werden, Betriebsparameter für den Betrieb des Messsystems beim Bestimmen des Analytmesswertes über die Fingerabdruck-Sensoreinrichtung festzulegen.

Bevorzugt sieht eine Fortbildung der Erfindung vor, dass die Steuereinrichtung weiterhin konfiguriert ist, bei der ergänzenden Auswertung der erfassten Fingerabdruck-Sensorsignale eine Fingergewebeanalyse durchzuführen und in Abhängigkeit hiervon Steuersignale für eine Benutzerinformation bereitzustellen.

Eine weitere bevorzugte Ausführungsform sieht eine Eingabeeinrichtung vor, die konfiguriert ist, einen Kodierungs- oder Kodeschlüssel zu empfangen, wobei die Steuereinrichtung weiterhin konfiguriert ist, zusätzlich den empfangenen Kodierungsschlüssel zu analysieren, um die Bestimmung des Analytmesswertes zu ermöglichen und / oder die Steuersignale zum Ausführen der weiteren Messsystemfunktionen zu liefern. In diesem Fall ist eine kombinierte Nutzung der Fingerabdruckidentifikation und einer oder mehrerer Kodierungsschlüssel ermöglicht. Die Kodierungsschlüssel können allgemein auch als Sicherheitsschlüssel bezeichnet werden. Ein solches System wird vorzugsweise in einer Multi-Betriebsart-Umgebung genutzt. Beispielsweise ist es einem Arzt, der sich autorisieren muss, nach einer Autorisierung für den Zugriff auf das System ermöglicht, ein vorinstalliertes Protokoll oder eine vorinstallierte Betriebart auszuwählen. Der Arzt kann einem ausgewählten Patienten dann erlauben, unter Nutzung dieses Protokolls oder dieser Betriebsart Daten zu erfassen. Auf diese Weise können Schritte zum Berichten über und zum Nutzen der Ergebnisse der Analyse dieser Daten auf einen Arzt und den Patienten begrenzt oder personalisiert werden. Optional kann nach einer Art Anonymisierung eine Meta-Analyse für einen Forscher ermöglicht werden, oder eine Übersichtsanalyse kann von einem Arzt für alle seine Patienten ausgeführt werden. In einer Ausführungsform kann eine Fingerabdrucksensoreinrichtung auf einem Datenerfassungs- oder -sammelgerät benutzt werden. Die Gerätesoftware und / oder eine optionale PC- oder Server-Software kann dann Regelfestlegungen umfassen, die für die Handhabung der erfassten Daten zusammen mit dem einen oder den mehreren Kodierungsschlüsseln nützlich ist. Zusätzliche Vorteile können sich aufgrund einer möglichen Integration einer Fingerabdrucksensoreinrichtung in ein optionales Kommunikationsgerät ergeben, das zur Erleichterung einer Datenübertragung und / oder eines Datenmanagements nutzbar ist. Eine solche Hardware-Konfiguration kann in verschiedenen Fällen genutzt werden. Beispielsweise kann sich ein Arzt an einem Einzelgerät einloggen oder anmelden und dieses Gerät dann für den Patienten vorbereiten. Alternativ ist ein von dem Arzt genutztes Gerät mit einem oder mehreren anderen Geräten gekoppelt. Eines der Geräte wird dann für eine Übergabe an den Patienten zur Datenerfassung oder -sammlung vorbereitet. Bei dem dem Arzt oder anderen medizinischen Personal zugeordneten Gerät kann es sich auch um ein Gerät handeln, welches nicht ein Datensammel- oder Datenerfassungsgerät ist. Es kann sich um ein angekoppeltes Kommunikationsgerät handeln, beispielsweise einen PC oder eine Kommunikationseinheit wie Accu-Check® SmartPix, welches einen Fingerabdrucksensor aufweist.

Das kodierungsschlüsselbasierte Zugriffsverfahren kann genutzt werden, um unterschiedliche Niveaus von Zugriffsrechten zu organisieren, die benötigt werden, um solche Multi-Betriebsart- und / oder Multi-Nutzer-Systeme zu organisieren. In einer Ausführungsform wird das System mittels eines Sicherheits- oder Kodierungsschlüssels geöffnet, worauf dann die Fingerabdruck-Sensoreinrichtung aktiviert wird. Ist es nachfolgend eigentlich notwendig, den Schlüssel anzugeben, wird anstelle einer langen Codesequenz die Fingerabdruck-Sensoreinrichtung genutzt.

In einem Multi-Betriebsart-System besteht Bedarf für einen ganzen Satz von "Schlüsseln". Die Fingerabdruck-Signale von verschiedenen Fingern können genutzt werden, um unterschiedliche Betriebsarten des Systems zu aktivieren. Beispielsweise kann nach einer Aktivierung mittels Eingabe eines Zahlenscodes ein Arzt seine Finger scannen. Das Bild des Zeigefingers wird genutzt, die Aktivierung des Arztes an einen Patienten für ein spezielles Protokoll zu übersenden. Hierdurch erhält der Patient die Rechte, die Messeinrichtung für dieses spezielle Protokoll zu nutzen. Der Arzt kann eine Editierfunktion für Protokolle oder Nutzungsszenarien auf dem Messsystem aktivieren, wenn er Bedingungen für ein Protokoll ändern möchte, um beispielsweise das Protokoll für einen speziellen Patienten anzupassen. Dieses spezielle Protokoll kann dann dem speziellen Patienten zugeordnet werden.

In Verbindung mit den Ausgestaltungen des Verfahrens zum Betreiben eines Messsystems zur Analytbestimmung, insbesondere Blutzuckerbestimmung, gelten die im Zusammenhang mit zugehörigen Ausführungsformen des Messsystems gemachten Erläuterungen entsprechend.

### Beschreibung bevorzugter Ausführungsbeispiele

Die Erfindung wird im Folgenden anhand von bevorzugten Ausführungsbeispielen unter Bezugnahme auf Figuren einer Zeichnung näher erläutert. Hierbei zeigen:
- Fig. 1: eine schematische Darstellung eines Messsystems zur Analytbestimmung in Mehrbenutzerausführung und
- Fig. 2: schematisch einen Ablauf beim Betrieb des Messsystems aus Fig. 1.

Auch wenn nachfolgend ein Ausführungsbeispiel anhand eines Messsystems für Blutzuckerbestimmungen aus Blutproben beschrieben wird, ist die Erfindung nicht hierauf beschränkt. Die vorgeschlagenen Technologien können ebenso für andere nachzuweisende Substanzen und Parameter sowie für andere Probenflüssigkeiten wie zum Beispiel Serum, Plasma, Urin, Speichel oder dergleichen angewandt werden, die hier zusammenfassend als "Analyten" bezeichnet werden. Als weitere Analyte im hier verstandenen Sinne kommen neben Glukose insbesondere Lactat, Cholesterin, Triglyzeride, Gerinnungsparameter wie die Prothrombinzeit (Quick-Wert) und dergleichen in Betracht.

Das Messsystem kann mit einem Server verbunden werden, von dem elektronische Daten empfangen werden können. Die elektronischen Daten können beliebige Betriebsaspekte des Messgerätes betreffen. Unterschiedliche elektronische Datensätze, die von dem Server empfangen werden, können in Abhängigkeit vom Ergebnis der Schlüssel- und / oder Fingerabdruck-Analyse angewendet werden.

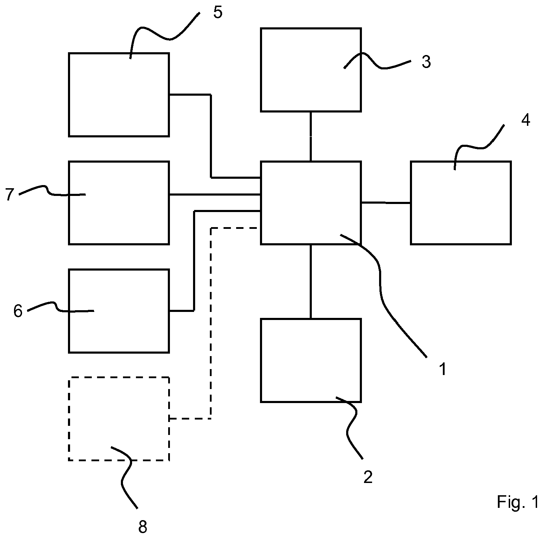

Fig. 1 zeigt eine schematische Darstellung eines Messsystems zur Blutzuckerbestimmung in Mehrbenutzerausführung, d. h. in einer Geräteausführung, die die Benutzung durch eine, bevorzugt durch mehrere unterschiedliche Personen ermöglicht. Hierfür ist das Messsystem mit Hardware- und Softwarekomponenten ausgeführt, was im Folgenden näher erläutert wird.

Bei dem Messsystem ist eine Steuereinrichtung 1 datentechnisch mit einer Analyseeinrichtung 2 verbunden. Mit Hilfe der Analyseeinrichtung 2 kann eine von einem Benutzer aufgegebene Blutprobe insbesondere hinsichtlich des Blutzuckerwertes analysiert werden. Eine solche Benutzung des Messsystems ist durch einen oder mehrere Benutzer nur dann möglich, wenn dieser zuvor im Rahmen einer Benutzeridentifizierung erkannt wurde. Für die Benutzeridentifizierung verfügt das Messsystem über eine Fingerabdruck-Sensoreinrichtung 3, die an die Steuereinrichtung 1 gekoppelt ist. Durch Auflegen eines oder mehrerer Finger auf die Fingerabdruck-Sensoreinrichtung 3 identifiziert sich ein Benutzer. Die Identifizierung ist erfolgreich, wenn die Steuereinrichtung 1 anhand in dem Messsystem hinterlegter elektronischer Daten einerseits und der Auswertung von erfassten Fingerabdruck-Sensorsignalen andererseits feststellt, dass die Fingerabdruck-Sensorsignale einem autorisierten Benutzer zugeordnet werden können. Nach einer solchen erfolgreichen Benutzeridentifizierung wird das Messsystem für die Analyse einer Blutprobe durch die Analyseeinrichtung 2 freigegeben.

Die im Rahmen der Analyse ermittelten Blutzuckerwerte werden in einer Speichereinrichtung 4 dem Benutzer zugeordnet abgelegt und / oder für eine Datenausgabe über eine Datenausgabeschnittstelle 5 bereitgestellt, sei es für einen kabelgebundene oder eine drahtlose Datenausgabe. Ergänzend oder alternativ kann eine Anzeige der Messwerte auf einem Display 6 erfolgen. Die Zuordnung der ermittelten Blutzuckerwerte zu dem identifizierten Benutzer erfolgt beispielsweise dadurch, dass dem Datensatz mit den Blutzuckermesswerten ein den identifizierten Benutzer angebender Bezeichner oder Identifizierer beigegeben wird.

Bei der in der Fig. 1 dargestellten Ausführungsform verfügt das Messsystem darüber hinaus über eine Probennahmeeinrichtung 7, die konfiguriert ist, dem Benutzer nach erfolgreicher Benutzeridentifizierung die Probennahme einer Blutprobe zu ermöglichen. Die genommene Blutprobe wird dann zur Analyseeinrichtung 2 transferiert und analysiert, zum Beispiel entlang eines Kapillarsystems.

Handelt es sich in einer anderen Ausführungsform um ein Messsystem zum Bestimmen eines anderen Analyten in einer Körperflüssigkeitsprobe, so sind insbesondere die Analyseeinrichtung 2 und die Probennahmeeinrichtung 7 entsprechend ausgelegt. Analyseeinrichtungen sowie Probennahmeeinrichtungen sind als solche in verschiedenen Ausgestaltungen bekannt, weshalb hier auf weitere Erläuterungen verzichtet werden kann.

Das Messsystem kann wahlweise eine oder mehrere weitere Gerätekomponenten 8 aufweisen, was in Fig. 1 mittels gestrichelter Linien gezeigt ist. Beispielsweise kann eine Eingabeeinrichtung vorgesehen sein, wobei die Eingabeeinrichtung konfiguriert ist, einen oder mehrere Kodierungsschlüssel zu empfangen. In einer bevorzugten Ausführungsform wird der Kodierungsschlüssel als Reaktion auf eine Nutzereingabe empfangen.

Die Steuereinrichtung 1 ist nun weiterhin konfiguriert, die über die Fingerabdruck-Sensoreinrichtung 3 erfassten Fingerabdruck-Sensorsignale über die Benutzeridentifizierung hinausgehend auszuwerten, um hieraus Steuersignale für andere Betriebsfunktionen des Messsystems bereitzustellen. Hierzu gehört beispielsweise das Ableiten von Steuersignalen für eine mit dem Display 6 gebildete Benutzerschnittstelle, die datentechnisch an die Steuereinrichtung 1 gekoppelt ist. Beispielsweise kann der Benutzer auf diese Weise über die Fingerabdruck-Sensoreinrichtung 3 in einem grafischen Benutzermenü navigieren und Funktionalitäten auswählen. Die Fingerabdruck-Sensoreinrichtung 3 ist in diesem Fall beispielsweise als eine Art Track-Pad-Vorrichtung ausgeführt.

Alternativ oder ergänzend kann die weitere Auswertung der Fingerabdruck-Sensorsignale mit Hilfe der Steuereinrichtung 1 vorsehen, dass Steuersignale in Abhängigkeit von Art oder Typ eines erkannten Fingers erzeugt werden, so dass zum Beispiel erste Steuersignale erzeugt werden, wenn der Zeigefinger erkannt wird, wohingegen zweite Steuersignale bereitgestellt werden, wenn ein anderer Finger der Hand des Benutzers beim ergänzenden Auswerten der Fingerabdruck-Sensorsignale erkannt wird. Die Steuereinrichtung 1 verfügt in diesem Fall über elektronische Informationen, die bestimmte Betriebsfunktionen des Messsystems in Abhängigkeit von der Fingerart zuordnen. Hierzu ist beispielsweise in der Speichereinrichtung 4 elektronische Information für eine Zuordnung zwischen Fingerart einerseits und Betriebsfunktionalität des Messsystems andererseits hinterlegt.

Alternativ oder ergänzend kann die Kodierungsschlüssel-Analyse zur Identifizierung unterschiedlicher Nutzer und / oder unterschiedlicher Betriebsarten genutzt werden, die verschiedenen Nutzern zugeordnet sind. Beispielsweise kann ein primärer Nutzer, beispielsweise ein Arzt oder anderes medizinisches Personal, mit Hilfe der Fingerabdruck-Sensoreinrichtung 3 identifiziert werden. Nach der Identifizierung des primären Nutzers wird das Messsystem generell für eine Betrieb freigegeben. Anschließend kann sich ein sekundärer Nutzer, zum Beispiel ein Patient, selbst mittels eines Kodierungsschlüssels identifizieren. Die Kodierungsschlüssel-Identifizierung kann das Messgerät zum Bestimmen eines speziellen Analyts herrichten oder freigeben.

Ergänzend oder alternativ ist die Steuereinrichtung 1 konfiguriert, bei der weitergehenden Auswertung der erfassten Fingerabdruck-Sensorsignale eine Fingergewebeanalyse durchzuführen und in Abhängigkeit hiervon Steuersignale für das Messsystem abzuleiten. So kann die ergänzende Analyse der Fingerabdruck-Sensorsignale ergeben, dass das Gewebe des Fingers, für den die Sensorsignale erfasst wurden, bereits in großem Umfang vernarbt ist, was die Folge einer häufigen Blutprobennahme an diesem Finger sein kann. Wird bei der ergänzenden Auswertung der Fingerabdruck-Sensorsignale ein solcher Umstand von der Steuereinrichtung erkannt, so werden Steuersignale erzeugt, die dem Benutzer über das Display 6 mitteilen, dass die Probennahme an einem anderen Finger empfohlen wird. Auch eine ergänzende oder alternative optische Signalisierung in Form einer farblichen Lampe kann in Abhängigkeit vom Ergebnis der Fingergewebeanalyse vorgesehen sein. So könnte eine rote Kontrollanzeige dem Benutzer des Messsystems signalisieren, dass der zunächst auf die Fingerabdruck-Sensoreinrichtung 3 aufgelegte Finger nicht für eine Probennahme geeignet ist.

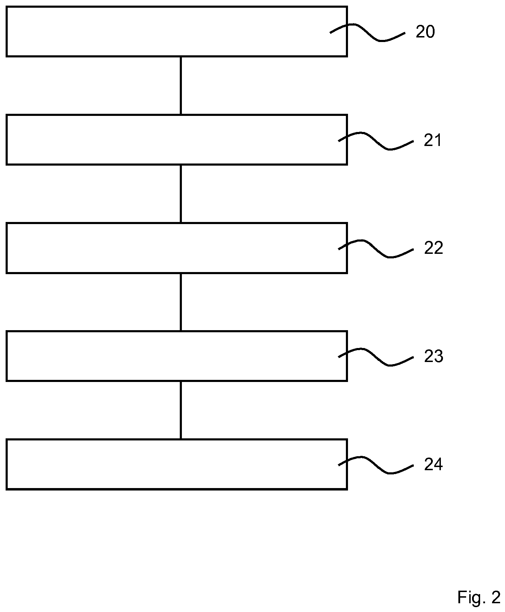

Fig. 2 zeigt zusammenfassend den Ablauf beim Betrieb des Messsystems aus Fig. 1 in einer beispielhaften Ausführung. Zunächst wird in einem Schritt 20 eine Benutzeridentifizierung durchgeführt. Ist die Benutzeridentifizierung erfolgreich, wird im Schritt 21 das Messsystem zur Benutzung durch den identifizierten Benutzer freigegeben. Im Schritt 22 werden dann die erfassten Fingerabdruck-Sensorsignale und / oder wahlweise zusätzlich erfasste Fingerabdruck-Sensorsignale über die Benutzeridentifizierung hinaus ausgewertet, um hieraus Steuersignale für weitere Messsystemfunktionen abzuleiten. Dieses erfolgt während und / oder nach einer Analyse einer Blutprobe durch die Analyseeinrichtung. Gemäß Fig. 2 wird bei dem gewählten Ausführungsbeispiel dann im Schritt 23 eine Blutprobe analysiert. Hierauf werden im Schritt 24 die ermittelten Blutzuckermesswerte für eine dem identifizierten Benutzer zugeordnete Datenspeicherung und / oder -ausgabe bereitgestellt.

Die in der vorstehenden Beschreibung, den Ansprüchen und der Zeichnung offenbarten Merkmale der Erfindung können sowohl einzeln als auch in beliebiger Kombination für die Verwirklichung der Erfindung in ihren verschiedenen Ausführungsformen von Bedeutung sein.

## Patentansprüche

1. Messsystem zur Analytbestimmung, mit:
- einer Analyseeinrichtung (2), die konfiguriert ist, einen Analytmesswert für eine aufgegebene Körperflüssigkeitsprobe zu bestimmen,
- einer Fingerabdruck-Sensoreinrichtung (3), die konfiguriert ist, Fingerabdruck-Sensorsignale für einen oder verschiedene Benutzer zu erfassen, und
- einer Steuereinrichtung (1), die datentechnisch mit der Analyseeinrichtung (2) und der Fingerabdruck-Sensoreinrichtung (3) verbunden ist,
wobei die Steuereinrichtung (1) konfiguriert ist, unter Verwendung erfasster Fingerabdruck-Sensorsignale eine Benutzeridentifizierung auszuführen und bei erfolgreicher Benutzeridentifizierung eine Analytmesswertbestimmung mit der Analyseeinrichtung (2) für einen identifizierten Benutzer freizugeben,
**dadurch gekennzeichnet, dass** die Steuereinrichtung (1) weiterhin konfiguriert ist,
- die erfassten Fingerabdruck-Sensorsignale und wahlweise weitere erfasste Fingerabdruck-Sensorsignale über die Benutzeridentifizierung hinaus ergänzend und frei von einer Berücksichtigung von elektronischen Informationen über die erfolgreiche Benutzeridentifizierung auszuwerten, um Steuersignale zum Ausführen von weiteren Messsystemfunktionen bereitzustellen,
- bei der ergänzenden Auswertung der erfassten Fingerabdruck-Sensorsignale ein oder mehrere Finger einer menschlichen Hand identifiziert werden und in Abhängigkeit von dem oder allen identifizierten Finger zugeordnete Steuersignale bereitzustellen und
- die ergänzende Auswertung der erfassten Fingerabdruck-Sensorsignale und das Bereitstellen der Steuersignalen während und / oder nach der Analytmesswertbestimmung für die aufgegebene Körperflüssigkeitsprobe durch die Analyseeinrichtung (2) auszuführen.

2. Messsystem nach Anspruch 1 **dadurch gekennzeichnet, dass** die Steuereinrichtung (1) weiterhin konfiguriert ist, bei erfolgreicher Benutzeridentifizierung Analytmesswerte, die mit Hilfe der Analyseeinrichtung (2) für die aufgegebene Körperflüssigkeitsprobe bestimmt werden, dem identifizierten Benutzer zugeordnet für eine Datenspeicherung und / oder -ausgabe bereitzustellen.

3. Messsystem nach Anspruch 1 oder 2, **dadurch gekennzeichnet, dass** die Steuereinrichtung (1) weiterhin konfiguriert ist, bei der ergänzenden Auswertung der erfassten Fingerabdruck-Sensorsignale in Abhängigkeit von den erfassten Fingerabdruck-Sensorsignalen Steuersignale zum Steuern eines Benutzermenüs bereitzustellen und das Benutzermenü den Steuersignalen entsprechend zu steuern.

4. Messsystem nach mindestens einem der vorangehenden Ansprüche, **gekennzeichnet durch** eine Eingabeeinrichtung, die konfiguriert ist, einen Kodierungsschlüssel zu empfangen, wobei die Steuereinrichtung (1) weiterhin konfiguriert ist, zusätzlich den empfangenen Kodierungsschlüssel zu analysieren, um die Bestimmung des Analytmesswertes zu ermöglichen und / oder die Steuersignale zum Ausführen der weiteren Messsystemfunktionen zu liefern.

5. Verfahren zum Betreiben eines Messsystems zur Analytbestimmung, bei dem eine Fingerabdruck-Sensoreinrichtung (3) und eine zur Analytmesswertbestimmung eingerichtete Analyseeinrichtung (2) zumindest datentechnisch mit einer Steuereinrichtung (1) verbunden sind, wobei das Verfahren die folgenden Schritte umfasst:
- Erfassen von Fingerabdruck-Sensorsignalen mittels der Fingerabdruck-Sensoreinrichtung (3),
- Auswerten der erfassten Fingerabdruck-Sensorsignale mittels der Steuereinrichtung (1), um einen Benutzer zu identifizieren,
- Freigeben einer Analytmesswertbestimmung für eine aufzugebende Körperflüssigkeitsprobe mit der Analyseeinrichtung (2) bei erfolgreicher Benutzeridentifizierung durch die Steuereinrichtung (1) und
- Ausführen einer Analytmesswertbestimmung für eine aufzugebende Körperflüssigkeitsprobe mit der Analyseeinrichtung (2),
**dadurch gekennzeichnet, dass** die Steuereinrichtung (1)
- Steuersignale zum Ausführen von weiteren Messsystemfunktionen bereitstellt, indem die erfassten Fingerabdruck-Sensorsignale und wahlweise weitere erfasste Fingerabdruck-Sensorsignale mittels der Steuereinrichtung (1) über die Benutzeridentifizierung hinaus ergänzend und frei von einer Berücksichtigung von elektronischen Informationen über die erfolgreiche Benutzeridentifizierung ausgewertet werden, wobei
- bei der ergänzenden Auswertung der erfassten Fingerabdruck-Sensorsignale ein oder mehrere Finger einer menschlichen Hand identifiziert werden und in Abhängigkeit von dem oder allen identifizierten Finger zugeordnete Steuersignale bereitgestellt werden und
- die ergänzende Auswertung der erfassten Fingerabdruck-Sensorsignale und das Bereitstellen der Steuersignalen während und / oder nach der Analytmesswertbestimmung für die aufgegebene Körperflüssigkeitsprobe durch die Analyseeinrichtung (2) ausgeführt werden.

6. Verfahren nach Anspruch 5, **dadurch gekennzeichnet, dass** bei erfolgreicher Benutzeridentifizierung Analytmesswerte, die mit Hilfe der Analyseeinrichtung (2) für eine aufgegebene Körperflüssigkeitsprobe zuvor bestimmt werden, mittels der Steuereinrichtung (1) dem identifizierten Benutzer zugeordnet für eine Datenspeicherung und / oder -ausgabe bereitgestellt werden.

7. Verfahren nach Anspruch 5 oder 6, **dadurch gekennzeichnet, dass** bei der ergänzenden Auswertung der erfassten Fingerabdruck-Sensorsignale in Abhängigkeit von den erfassten Fingerabdruck-Sensorsignalen Steuersignale zum Steuern eines Benutzermenüs bereitgestellt werden und das Benutzermenü den Steuersignalen entsprechend gesteuert wird.

8. Verfahren nach mindestens einem der Ansprüche 5 bis 7, **dadurch gekennzeichnet, dass** von einer Eingabeeinrichtung ein Kodierungsschlüssel empfangen wird und die Steuereinrichtung (1) zusätzlich den empfangenen Kodierungsschlüssel analysiert, um die Bestimmung des Analytmesswertes freizugeben und / oder die Steuersignale zum Ausführen der weiteren Messsystemfunktionen zu liefern.

## Claims

1. A measurement system for analyte determination, having:
- an analytical device (2) which is configured to determine an analyte measurement value for a presented bodily fluid specimen,
- a fingerprint sensor device (3) which is configured to record fingerprint sensor signals for one or more users, and
- a control device (1) which is data-linked to the analytical device (2) and to the fingerprint sensor device (3),
wherein the control device (1) is configured
- to carry out a user identification using recorded fingerprint sensor signals and, upon successful user identification, to enable a determination, using the analytical device (2), of an analyte measurement value for an identified user, and **characterized in that** the control device is furthermore configured
- to evaluate the recorded fingerprint sensor signals and optionally additional recorded fingerprint sensor signals in addition to the user identification and independently of taking into account electronic information regarding successful user identification, in order to provide control signals for carrying out additional measurement system functions,
- to identify one or more fingers of a human hand during the additional evaluation of the recorded fingerprint sensor signals and to provide control signals allocated as a function of the identified finger or all identified fingers,
- to carry out the additional evaluation of the recorded fingerprint sensor signals and to provide the control signals during and/or after the analyte measurement value determination for the presented bodily fluid specimen by means of the analytical device (2).

2. The measurement system as claimed in claim 1, **characterized in that** the control device (1) is further configured to provide, upon successful user identification, analyte measurement values for the presented bodily fluid specimen which are determined with the assistance of the analytical device (2) and allocated to the identified user, for data storage and/or output.

3. The measurement system as claimed in claim 1 or claim 2, **characterized in that** the control device (1) is further configured to provide control signals for controlling a user menu during the additional evaluation of the recorded fingerprint sensor signals as a function of the recorded fingerprint sensor signals and to control the user menu in accordance with the control signals.

4. The measurement system as claimed in one of the preceding claims, **characterized by** an input device which is configured to receive an encryption key, wherein the control device (1) is further configured to additionally analyse the received encryption key, in order to permit the determination of the analyte measurement value and/or to deliver the control signals for carrying out the additional measurement system functions.

5. A method for operating a measurement system for analyte determination, in which a fingerprint sensor device (3) and an analytical device (2) equipped for the determination of an analyte measurement value are at least data-linked to a control device (1), wherein the control device carries out the following steps:
- recording fingerprint sensor signals by means of the fingerprint sensor device (3),
- evaluating the recorded fingerprint sensor signals by means of the control device (1) in order to identify a user,
- upon successful user identification by means of the control device (1), enabling a determination, with the analytical device (2), of an analyte measurement value for a bodily fluid specimen to be presented, and
- carrying out a determination, with the analytical device (2), of an analyte measurement value for a bodily fluid specimen to be presented,
**characterized in that** the control device (1) provides control signals for carrying out additional measurement system functions, in which the recorded fingerprint sensor signals and optionally additional recorded fingerprint sensor signals are evaluated by means of the control device (1) in addition to the user identification and independently of taking into account electronic information regarding the successful user identification, wherein
- one or more fingers of a human hand are identified during the additional evaluation of the recorded fingerprint sensor signals, and control signals are provided which are allocated as a function of the identified finger or all the identified fingers, and
- the additional evaluation of the recorded fingerprint sensor signals and the provision of the control signals during and/or after the analyte measurement value determination for the presented bodily fluid specimen are carried out by means of the analytical device (2).

6. The method as claimed in claim 5, **characterized in that**, following successful user identification, analyte measurement values for a presented bodily fluid specimen which have previously been determined with the assistance of the analytical device (2) are allocated to the identified user by means of the control device (1), for data storage and/or output.

7. The method as claimed in claim 5 or claim 6, **characterized in that** control signals are provided for controlling a user menu during the additional evaluation of the recorded fingerprint sensor signals as a function of the recorded fingerprint sensor signals, and the user menu is controlled in accordance with the control signals.

8. The method as claimed in at least one of claims 5 to 7, **characterized in that** an encryption key is received by an input unit and the control device (1) additionally analyses the received encryption key for enabling the determination of the analyte measurement value and/or for providing the control signals for carrying out the additional measurement system functions.

## Revendications

1. Système de mesure, destiné à déterminer des analytes, comprenant :
- un dispositif d'analyse (2), qui est configuré pour déterminer une valeur de mesure d'analytes pour un échantillon de fluide corporel appliqué,
- un dispositif capteur d'empreintes digitales (3), qui est configuré pour détecter des signaux de capteur d'empreintes digitales pour un ou pour plusieurs utilisateurs et
- un dispositif de commande (1), qui est connecté par technique de données avec le dispositif d'analyse (2) et le dispositif capteur d'empreintes digitales (3),
le dispositif de commande (1) étant configuré pour,
- en utilisant des signaux de capteur d'empreintes digitales détectés, procéder à une identification de l'utilisateur et si l'identification de l'utilisateur a abouti, pour valider une détermination de la valeur de mesure d'analytes à l'aide du dispositif d'analyse (2) pour un utilisateur identifié et
**caractérisé en ce que** le dispositif de commande est configuré en outre
- pour évaluer complémentairement les signaux de capteur d'empreintes digitales détectés et facultativement d'autres signaux de capteur d'empreintes digitales détectés, au-delà de l'identification de l'utilisateur et sans considération d'informations électroniques sur l'aboutissement de l'identification de l'utilisateur, pour mettre à disposition des signaux de commande pour la réalisation de fonctions additionnelles du système de mesure,
- lors de l'évaluation complémentaire des signaux de capteur d'empreintes digitales détectés, pour identifier plusieurs doigts d'une main humaine et en fonction d'un ou de tous les doigts identifiés, pour mettre à disposition des signaux de commande associés et
- pour procéder à l'évaluation complémentaire des signaux de capteur d'empreintes digitales détectés et à la mise à disposition des signaux de commande pendant et/ou après la détermination de la valeur de mesure d'analytes pour l'échantillon de fluide corporel appliqué par le dispositif d'analyse (2).

2. Système de mesure selon la revendication 1, **caractérisé en ce que** le dispositif de commande (1) est configuré en outre pour, lors de l'aboutissement de l'identification de l'utilisateur, mettre à disposition des valeurs de mesure d'analytes, qui sont déterminées à l'aide du dispositif d'analyse (2) pour l'échantillon de fluide corporel appliqué, associées à l'utilisateur identifié pour une mémorisation et/ou une édition de données.

3. Système de mesure selon la revendication 1 ou 2, **caractérisé en ce que** le dispositif de commande (1) est configuré en outre pour mettre à disposition, lors de l'évaluation complémentaire des signaux de capteur d'empreintes digitales détectés, en fonction des signaux détectés par le capteur d'empreintes digitales des signaux de commande destinés à commander un menu utilisateur et pour commander le menu utilisateur en conséquence des signaux de commande.

4. Système de mesure selon au moins l'une quelconque des revendications précédentes, **caractérisé par** un dispositif de saisie qui est configuré pour réceptionner une clé de codage, le dispositif de commande (1) étant configuré en outre pour analyser en supplément la clé de codage réceptionnée, pour permettre de déterminer la valeur de mesure d'analyte et/ou pour fournir les signaux de commande pour la réalisation des fonctions additionnelles du système de mesure.

5. Procédé, destiné à faire fonctionner un système de mesure destiné à déterminer des analytes, lors duquel, un dispositif de capteur d'empreintes digitales (3) et un dispositif d'analyse (2) aménagé pour la détermination de valeurs de mesure d'analytes sont connectés au moins par technique de données avec un dispositif de commande (1), le dispositif de commande réalisant les étapes suivantes consistant à :
- détecter des signaux de capteur d'empreintes digitales à l'aide du dispositif capteur d'empreintes digitales (3),
- évaluer les signaux de capteur d'empreintes digitales détectés à l'aide du dispositif de commande (1), pour identifier un utilisateur,
- valider une détermination de valeurs de mesure d'analyte pour un échantillon de fluide corporel qui doit être appliqué, à l'aide du dispositif d'analyse (2) en cas d'aboutissement de l'identification de l'utilisateur à l'aide du dispositif de commande (1), et
- procéder à une détermination de valeurs de mesure d'analytes pour un échantillon de fluide corporel qui doit être appliqué, à l'aide du dispositif d'analyse (2),
**caractérisé en ce que** le dispositif de commande (1) met à disposition des signaux de commande (1) pour la réalisation de fonctions additionnelles du système de mesure, **en ce que** les signaux de capteur d'empreintes digitales détectés et facultativement d'autres signaux de capteur d'empreintes digitales détectés sont évalués à l'aide du dispositif de commande (1) au-delà de l'identification de l'utilisateur et sans considération d'informations électroniques concernant l'aboutissement de l'identification de l'utilisateur,
- lors de l'évaluation complémentaire des signaux de capteur d'empreintes digitales détectés, un ou plusieurs doigts d'une main humaine étant identifiés et en fonction du ou de tous les doigts identifiés, des signaux de commande associés étant mis à disposition, et
- l'évaluation complémentaire des signaux de capteur d'empreintes digitales détectés et la mise à disposition des signaux de commande étant réalisées par le dispositif d'analyse (2) pendant et/ou après la détermination de valeurs de mesure d'analytes pour l'échantillon de fluide corporel appliqué.

6. Procédé selon la revendication 5, **caractérisé en ce que** lors de l'aboutissement de l'identification de l'utilisateur, des valeurs de mesure d'analytes qui sont préalablement déterminées à l'aide du dispositif d'analyse (2) pour un échantillon de fluide corporel appliqué sont associées à l'utilisateur identifié à l'aide du dispositif de commande (1) pour une mémorisation et/ou une édition de données.

7. Procédé selon la revendication 5 ou 6, **caractérisé en ce que** lors de l'évaluation complémentaire des signaux de capteur d'empreintes digitales, en fonction des signaux de capteur d'empreintes digitales détectés, des signaux de commande sont mis à disposition pour commander un menu utilisateur et le menu utilisateur est commandé en conséquence des signaux de commande.

8. Procédé selon au moins l'une quelconque des revendications 5 à 7, **caractérisé en ce que** une clé de codage est réceptionnée par un dispositif de saisie et le dispositif de commande (1) analysant en supplément la clé de codage réceptionnée, pour valider la détermination de la valeur de mesure d'analytes et/ou pour fournir les signaux de commande pour la réalisation des fonctions additionnelles de système de mesure.
